# EUROPÄISCHE PATENTSCHRIFT

(11) **EP 3 756 696 B1**
(45) Veröffentlichungstag und Bekanntmachung des Hinweises auf die Patenterteilung: **03.04.2024**
(21) Anmeldenummer: 20179939.2
(22) Anmeldetag: 15.06.2020
(51) Int. Cl.: A61L 9/14, A01M 1/20, B05B 17/06

(54) **PIEZOELEKTRISCH BETRIEBENER DISPENSER ZUM AUSTRAGEN FLÜSSIGER BZW. FLÜCHTIGER SUBSTANZEN**
PIEZOELECTRICALLY OPERATED DISPENSER FOR DISPENSING LIQUID OR VOLATILE SUBSTANCES
DISTRIBUTEUR À COMMANDE PIÉZOÉLECTRIQUE PERMETTANT DE DISTRIBUER DU LIQUIDE OU DES SUBSTANCES VOLATILES

(30) Priorität: 26.06.2019 DE 102019117212
(43) Veröffentlichungstag der Anmeldung: 30.12.2020
(73) Patentinhaber: CTR, Lda, 2135-301 Samora Correia (PT)
(72) Erfinder: Vieira, Pedro Queiroz, 2605-197 Belas (PT); Bual, Miguel do Canto Moniz, 1400-085 Lissabon (PT)
(74) Vertreter: Liebl, Thomas

(56) Entgegenhaltungen:
- EP-A2- 0 897 755
- WO-A1-2006/004891
- WO-A1-2013/159142
- WO-A2-2005/106344
- WO-A2-2008/008459
- WO-A2-2008/076326
- WO-A2-2015/175527
- US-A1- 2018 043 048

## Beschreibung

Die Erfindung betrifft einen piezoelektrisch betriebenen Dispenser zum Austragen flüssiger bzw. flüchtiger Substanzen nach dem Oberbegriff des Anspruchs 1.

Dispenser dienen generell zum Verteilen eines flüssigen Wirkstoffes, wie beispielsweise eines Parfums, eines Insektizides oder einer anderen Substanz. Hierbei ist es weiter allgemein bekannt, die Substanzen mittels eines piezoelektrisch betriebenen Dispensers, der ein piezoelektrisch betriebenes Zerstäubersystem aufweist, in feine bzw. feinste Teilchen oder Tröpfchen zu überführen und als Sprühnebel an die Umgebung abzugeben. Unter einem piezoelektrisch betriebenen Zerstäubersystem wird dabei ein Zerstäubersystem mit einem Piezoelement verstanden, das den Piezoeffekt ausnutzt, um zum Beispiel durch Anlegen einer elektrischen Spannung eine mechanische Bewegung oder Schwingung auszuführen (Piezoaktor), mittels welcher mechanischen Bewegung oder Schwingung dann die Tröpfchen erzeugt werden. Dies ist allgemein bekannt, und muss nicht weiter erläutert werden.

Die mittels des Dispensers auszutragenden flüssigen bzw. flüchtigen Substanzen werden dabei regelmäßig in einem Behältnis vorgehalten, das in ein Gehäuse des Dispensers eingesetzt wird, um die Substanz über zum Beispiel ein Dochtsystem zum piezoelektrisch betriebenen Zerstäubersystem zu führen. Die die Substanzen aufweisenden Behältnisse der piezoelektrisch betriebenen Dispenser weisen ein Gehäuse auf, in dem das Behältnis vollständig aufgenommen ist, so dass für das Einsetzen und auch für die Entnahme der Behältnisse jeweils das Gehäuse des Dispensers zu öffnen ist. Dies ist relativ arbeitsintensiv und auch zeitaufwendig.

Die WO 2006/004891 A1 offenbart eine Vorrichtung zur Abgabe einer flüchtigen Substanz mit einer Kartusche zur Aufnahme einer Vielzahl von Behältern, die jeweils eine flüchtige Substanz enthalten. Die Kartusche ist drehbar gelagert, so dass jeder der Behälter in eine Eingriffsposition gebracht werden kann, in der die flüchtige Substanz abgegeben werden kann. Das Gehäuse ist an der Unterseite komplett offen ausgebildet, um die einzelnen Behälter in die Kartusche einzusetzen bzw. auszutauschen. Die Vorrichtung kann ein piezoelektrisch betätigter Flüssigkeitszerstäuber vom Vibrationstyp sein.

Die WO 2008/0084592 offenbart eine Diffusionsvorrichtung mit einem Gehäuse, in dem eine Batterie angeordnet ist und das zur Aufnahme eines austauschbaren Fluidreservoirs zur Aufnahme eines Fluids geeignet ist, wobei das Fluidreservoir einen Docht zur Bewegung des Fluids zu einem Auslassende desselben aufweist. Die Diffusionsvorrichtung umfasst ferner ein piezoelektrisches Element, das von einer Stromquelle zum Vibrieren einer perforierten Öffnungsplatte erregt wird, die neben dem Auslassende des Dochts angeordnet ist, wobei das piezoelektrische Element eine Vibrationsbewegung entwickelt, um das Fluid vom Auslassende durch die perforierte Auslassplatte und in die Atmosphäre zu pumpen. Eine das Gehäuse auf der Unterseite verschleißende untere Grundplatte enthält ein Scharnier, das eine Tür definiert, die nach unten weggeschwenkt werden kann, um Zugang zu einem inneren Teil der Diffusionsvorrichtung zu erhalten. Das Abklappen ist erst möglich, wenn eine Schraube gelöst wird, mit der die Tür am Gehäuse gehaltert ist.

Demgegenüber ist es Aufgabe der vorliegenden Erfindung, einen piezoelektrisch betriebenen Dispenser zum Austragen flüssiger bzw. flüchtiger Substanzen vorzusehen, mittels dem das Einsetzen und der Austausch der die Substanzen aufnehmenden Behältnisse wesentlich vereinfacht ist und vom Bediener innerhalb kürzester Zeit durchgeführt werden kann.

Diese Aufgabe wird gelöst mit den Merkmalen des Anspruchs 1. Vorteilhafte Ausgestaltungen sind Gegenstand der darauf rückbezogenen Unteransprüche.

Gemäß Anspruch 1 wird ein piezoelektrisch betriebener Dispenser zum Austragen flüssiger bzw. flüchtiger Substanzen vorgesehen, der ein Gehäuse mit einem Gehäuseinnenraum aufweist. Ferner umfasst der piezoelektrisch betriebene Dispenser ein Behältnis, das lösbar mit dem Gehäuse verbindbar ist und in dem eine auszutragende und/oder zu verflüchtigende Substanz aufgenommen ist. Ferner umfasst der piezoelektrische Dispenser ein piezoelektrisch betriebenes Zerstäubersystem, das in dem Gehäuseinnenraum angeordnet ist und mittels dem die aus dem Behältnis entnommene Substanz zerstäubt wird. Dabei ist das Behältnis im montierten Zustand im Wesentlichen vollständig, insbesondere vollständig, im Gehäuseinnenraum aufgenommen und dort mittels wenigstens einer Schnappverbindung lösbar gehaltert. Das Gehäuse weist eine Einführöffnung für das Behältnis auf, über die das Behältnis in den Gehäuseinnenraum einführbar ist. Das Gehäuse weist weiter, vorzugsweise im Bereich der Einführöffnung, wenigstens eine Fingereingriffsöffnung auf, dergestalt, dass beim Einführen des Behältnisses in den Gehäuseinnenraum und beim Entnehmen des Behältnisses aus dem Gehäuseinnenraum ein Fingereingriff in den Gehäuseinnenraum möglich ist.

Mit einem derartigen Aufbau eines piezoelektrischen Dispensers wird eine wesentliche Vereinfachung im Hinblick auf das Einsetzen und die Entnahme eines im Wesentlichen vollständig bzw. vorzugsweise vollständig im Gehäuseinnenraum eines piezoelektrischen Dispensers aufgenommenen Behältnisses erzielt, da über die wenigstens eine Fingereingriffsöffnung sichergestellt ist, dass das Einsetzen und die Entnahme schnell und funktionssicher von Hand bzw. mit den Fingern erfolgen kann. Es wird somit ein geschlossener Dispenser mit einem Zugang zum Behältnis von außen zur Verfügung gestellt, der das direkte Einführen bzw. Austauschen des Behältnisses in den Dispenser von außen her erlaubt, und zwar ohne Demontage des Gehäuses und ohne Öffnen von Abdeckungen des Gehäuses.

Die Schnappverbindung stellt dabei sicher, dass die Behältnisse im eingesetzten, montierten Zustand zuverlässig und funktionssicher in ihrer gewünschten Position gehalten werden. Die Schnappverbindung stellt andererseits aber auch eine solche lösbare Verbindung zur Verfügung, die einfach, zum Beispiel durch Aufbringen einer entsprechenden Kraft (zum Beispiel einer Zugkraft), überdrückt werden kann, um die Halterung zu lösen und das Behältnis aus dem Gehäuseinnenraum zu entnehmen. Dies erfolgt durch ein einfaches Ergreifen des Behältnisses mittels der Finger im Gehäuseinnenraum. Entsprechendes gilt für das Einsetzen eines Behältnisses von Hand bzw. mit den Fingern, bei dem die Schnappverbindung in analoger Weise solange überdrückt wird, bis das Behältnis wieder funktionssicher im Gehäuseinnenraum verrastet ist.

Grundsätzlich sind dabei sämtliche Arten von Schnappverbindungen möglich, zum Beispiel auch eine zylindrische bzw. zylinderförmige Schnappverbindung, bei der ein elastisch rückfedernder Ringwulst einen behältnisseitigen Haltebereich hintergreift oder eine kugelige Schnappverbindung, bei der eine elastisch rückfedernde kugelige Überdeckungsfläche einen kugeligen, behältnisseitigen Haltebereich hintergreift. Besonders bevorzugt ist jedoch eine Ausgestaltung, bei der die Schnappverbindung durch wenigstens einen, im Gehäuseinnenraum elastisch rückfedernd angeordneten Schnapphaken gebildet ist, der wenigstens einen, vorzugsweise endseitig angeordneten, Rastvorsprung aufweist, der bei einem in das Gehäuse eingesetzten, montierten Behältnis einen behältnisseitigen Haltebereich hintergreift oder in einen behältnisseitigen Haltebereich eingreift, so dass das Behältnis lösbar im Gehäuseinnenraum gehaltert ist. Dabei werden die Schnapphaken beim Einführen des Behältnisses kurzzeitig ausgelenkt und rasten dann am Ende der Montage entsprechend ein. Beim Demontieren werden die Schnapphaken nach Überwinden einer bestimmten Haltekraft ebenfalls wieder ausgelenkt bzw. überdrückt, wodurch die Schnapphaken bzw. deren Rastvorsprung außer Eingriff mit dem Haltebereich gelangen und dementsprechend die Schnappverbindung gelöst wird.

Eine derartige Schnappverbindung weist den Vorteil auf, dass sie besonders einfach in der Herstellung ist und zudem eine hohe Funktionssicherheit aufweist. Letzteres nicht zuletzt dadurch, dass insbesondere bei einer Schnappverbindung mit Schnapphaken der Hinterschnittwinkel über die Geometrie des Rastvorsprungs des Schnapphakens auf besonders einfache Weise festgelegt und damit die Festigkeit der lösbaren Verbindung zuverlässig eingestellt werden kann.

Gemäß einer besonders bevorzugten konkreten Ausgestaltung weist das Behältnis einen in der Art eines Rohrstutzens und/oder in der Art eines Flaschenhalses ausgebildeten Auslassöffnungsbereich auf, der an seiner Außenseite einen wenigstens abschnittsweise, vorzugsweise ringförmig, umlaufenden Wulst oder eine wenigstens abschnittsweise, vorzugsweise ringförmig, umlaufende Nut, als Haltebereich aufweist. Ein derartig ausgebildetes Behältnis lässt sich auf besonders einfache Art und Weise funktionssicher mittels der erfindungsgemäßen Schnappverbindung lösbar im Gehäuseinnenraum des Dispensers haltern.

Besonders bevorzugt ist weiter eine Ausgestaltung, bei der im Gehäuseinnenraum eine Mehrzahl von Schnapphaken vorgesehen ist, die im eingesetzten Zustand des Behältnisses ringförmig und voneinander beabstandet um den Auslassöffnungsbereich des Behältnisses herum angeordnet sind und mit ihren jeweiligen Rastvorsprüngen den Wulst hintergreifen oder in die Nut eingreifen. Mit einer derartigen Mehrzahl von Schnapphaken wird eine besonders funktionssichere Schnappverbindung zur Verfügung gestellt, die es erlaubt, den Behälter zuverlässig und funktionssicher in der gewünschten Position zu halten. Des Weiteren kann es hier dann zu keinen unerwünschten Verkippungen und Verkantungen kommen.

Gemäß einer weiteren besonders bevorzugten Ausgestaltung ist im Gehäuseinnenraum eine innere Gehäuseschale vorgesehen, die einen im Gehäuseinnenraum ausgebildeten Aufnahmeraum für das Behältnis ausbildet, insbesondere begrenzt. Eine derartige innere Gehäuseschale gewährleistet eine erhöhte konstruktive Flexibilität, insbesondere in Verbindung mit der Anordnung von Bauteilen bzw. der Ausbildung von Bauelementen im Gehäuseinnenraum. Beispielsweise kann alternativ oder zusätzlich an der inneren Gehäuseschale auch der gehäuseseitige Teilbereich der Schnappverbindung angeordnet und/oder ausgebildet sein, vorzugsweise die Schnapphaken der Schnappverbindung angeordnet und/oder ausgebildet sein.

Das Zerstäubersystem selbst weist bevorzugt ein elektrisch betätigbares Piezoelement auf. Weiter weist das Zerstäubersystem eine Zuführeinrichtung auf, mittels der die in dem Behälter aufgenommene Substanz dem Piezoelement zuführbar ist. Die Zuführeinrichtung kann hier zum Beispiel durch ein separates Bauteil gebildet sein, zum Beispiel durch einen Docht oder eine Pumpe, das zum Beispiel mit dem Behältnis verbindbar ist.

Besonders vorteilhaft ist hierbei eine Ausführungsform, bei der die innere Gehäuseschale einen sich in Hochachsenrichtung von dem eingesetzten, montierten Behältnis weg erstreckenden Dom aufweist, der mit seinem unteren Teilbereich den gehäuseseitigen Teilbereich der Schnappverbindung ausbildet, vorzugsweise die Schnapphaken der Schnappverbindung ausbildet. Weiter kann bei dieser Ausführungsvariante das Piezoelement bevorzugt in einem sich an den unteren Teilbereich des Doms anschließenden oberen Teilbereich des Doms angeordnet und/oder aufgenommen sein. Hierbei ist es weiter vorteilhaft, wenn das Piezoelement in einen, in Hochachsenrichtung gesehen, oberen Gehäusebereich des Gehäuses mündet, der eine Auslassöffnung, bevorzugt einen Auslasskanal, höchst bevorzugt einen in der Art eines Kamins ausgebildeten Auslasskanal, für die vom Piezoelement zerstäubte, abzugebende Substanz aufweist und/oder ausbildet. Mit einem derartigen Aufbau wird insbesondere eine kompakte und wenig bauteilintensive Bauweise erzielt, die höchsten Anforderungen an Dispenser mit einem hohen Wirkungsgrad genügt.

Die Zuführeinrichtung kann grundsätzlich auf unterschiedliche Weise ausgebildet sein, so zum Beispiel durch ein Pumpsystem, bei dem die dem Piezoelement zuzuführende Substanz bzw. Flüssigkeit aus dem Behältnis abgepumpt wird. Besonders bevorzugt ist die Zuführeinrichtung jedoch durch einen Docht gebildet, der im eingesetzten, montierten Zustand des Behältnisses mit einem ersten Teilbereich in die im Behältnis aufgenommene Substanz einragt und mit einem gegenüberliegenden freien Ende an das Piezoelement angrenzt, vorzugsweise aus dem Behältnis ragt und an das Piezoelement angrenzt. Der Docht kann hier aus unterschiedlichsten Materialien hergestellt sein, so zum Beispiel aus einem Fasergewebe oder aber auch zum Beispiel durch eine Keramik. Mit einem derartigen Docht wird die Substanz bzw. Flüssigkeit aus dem Behältnis über die Kapillarwirkung nach oben zum freien Dochtende gefördert und dort dem Piezoelement zugeführt. Dabei kann der Docht beispielsweise das Piezoelement direkt berühren. Vorzugsweise erfolgt die Substanz- bzw. Flüssigkeitsabgabe an eine Lochplattenanordnung des Piezoelementes. Bei einer Betätigung des Piezoelementes kommt es dann zum Schwingen dieser Lochplattenanordnung, was dann wiederum zum Zerstäuben der Substanz bzw. der Flüssigkeit führt.

Das Gehäuse selbst weist bevorzugt eine ein- oder mehrteilig ausgebildete Außenschale auf, die den Gehäuseinnenraum nach außen hin begrenzt, einen Boden- oder Aufstandsbereich ausbildet und wenigstens eine Auslassöffnung für die abzugebende Substanz aufweist. Besonders bevorzugt ist hierbei eine Ausführungsform, bei der das Gehäuse eine zweiteilige Außenschale mit einer, bezogen auf die Hochachsenrichtung, unteren, den Boden- oder Aufstandsbereich ausbildenden Gehäuseaußenschale und einer damit verbundenen oberen, die wenigstens eine Auslassöffnung aufweisenden Gehäuseschale aufweist. Ein derartiger mehrteiliger Aufbau eröffnet größere designtechnische Möglichkeiten bei der Gestaltung der Gehäuseaußenschale bzw. einen grundsätzlich einfacheren Zugang zum Gehäuseinnenraum, wenn dies beispielsweise in Verbindung mit einer Steuerungselektronik erforderlich sein sollte.

Gemäß einer weiteren besonders bevorzugten Ausgestaltung bildet die Einführöffnung für das Behältnis gleichzeitig die wenigstens eine Fingereingriffsöffnung mit aus. Dies kann beispielsweise einfach dadurch erfolgen, dass die Einführöffnung zur Ausbildung der wenigstens einen Fingereingriffsöffnung mit einem Übermaß gegenüber der Außenumfangsgeometrie des Behältnisses ausgebildet ist. Ein derartiger Aufbau mit einer derartigen gleichzeitig die wenigstens eine Fingereingriffsöffnung mit ausbildenden Einführöffnung lässt sich auf technisch besonders einfache Weise erzielen und herstellen.

Erfindungsgemäß sind die Einführöffnung und zwei Fingereingriffsöffnungen in dem Boden- oder Aufstandsbereich der Außenschale des Gehäuses ausgebildet. Damit kann das Behältnis einfach von der Boden- bzw. Unterseite der Außenschale des Gehäuses her in den Gehäuseinnenraum eingesetzt bzw. aus diesem entnommen werden.

Die wenigstens eine Fingereingriffsöffnung kann dabei zum Beispiel grundsätzlich als komplett um die Einführöffnung umlaufende Fingereingriffsöffnung ausgebildet sein, also zum Beispiel durch eine einzige große und damit gleichzeitig die Fingereingriffsöffnung als auch die Einführöffnung ausbildende Öffnung mit einem solchen Übermaß gegenüber der Außenumfangsgeometrie des Behältnisses ausgebildet sein, dass beispielsweise mit allen Fingern einer Hand in den Gehäuseinnenraum eingegriffen werden kann. Alternativ dazu kann die Einführöffnung aber auch zwischen mehreren anschließenden, vorzugsweise zwischen zwei auf gegenüberliegenden Seiten anschließenden, Fingereingriffsöffnungen liegen. Das heißt, dass grundsätzlich auch mehr als zwei, das heißt zum Beispiel auch fünf, Fingereingriffsöffnungen vorgesehen sein können, so dass mit mehr als zwei Fingern in den Gehäuseinnenraum eingegriffen werden kann. Erfindungsgemäß ist dabei vorgesehen, dass die Einführöffnung in einem mittleren Bereich oder in etwa mittig zwischen den zwei sich daran auf gegenüberliegenden Seiten hin anschließenden Fingereingriffsöffnungen liegt. Weiter kann vorgesehen sein, dass die Einführöffnung mitsamt den zwei Fingereingriffsöffnungen eine langgestreckte Schlitzausnehmung ausbildet. Ein derartiger Aufbau ist besonders vorteilhaft, weil hier dann beispielsweise zu beiden Seiten eines eingesetzten Behältnisses (beispielsweise mit dem Daumen und Zeigefinger bzw. mit dem Daumen und Mittelfinger einer Hand) einfach in das Innere des Gehäuses gegriffen und das Behältnis zum Herausnehmen und Lösen bzw. Überdrücken der Schnappverbindung im Gehäuseinnenraum ergriffen werden kann. Analoges gilt entsprechend für das Einsetzen des Behältnisses in den Gehäuseinnenraum.

Eine derartige, vorzugsweise langgestreckte, Schlitzausnehmung ist insbesondere auch in Verbindung mit einer Anordnung derselben in dem Boden- oder Aufstandsbereich der Außenschale des Gehäuses vorteilhaft, weil dann über diesen Bodenbereich ein einfaches Einsetzen möglich ist und zudem im Funktionszustand keine Sichtbarkeit der Schlitzausnehmung gegeben ist.

Besonders bevorzugt ist des Weiten ein Aufbau, bei dem die Schlitzausnehmung in dem zwischen den beiden Fingereingriffsöffnungen liegenden und die Einführöffnung für das Behältnis ausbildenden Ausnehmungsbereich eine Markierung und/oder eine dem Außenumfang des Behältnisses entsprechende Verbreiterung oder Einschnürung aufweist. Damit wird eine einfache Zuordnung bzw. Markierung der Einführöffnung erzielt, so dass eine Fehlbedienung funktionssicher ausgeschlossen wird.

Es versteht sich, dass die wenigstens eine gehäuseseitig ausgebildete Fingereingriffsöffnung zum Gehäuseinnenraum hin in einen Fingereingriffsraum übergeht.

Weiter ist bevorzugt vorgesehen, dass sich die innere Gehäuseschale, der Geometrie der Schlitzausnehmung folgend, mit einem unteren Gehäuseschalenbereich turmartig an die Schlitzausnehmung anschließt und gleichzeitig den Fingereingriffsraum sowie den Behältnis-Aufnahmeraum ausbildet. Dabei ist bevorzugt vorgesehen, dass sich der untere Gehäuseschalenbereich der inneren Gehäuseschale so weit in Hochachsenrichtung nach oben erstreckt, dass das Behältnis im eingesetzten Zustand im Wesentlichen vollständig im Gehäuseinnenraum und/oder im Behältnis-Aufnahmeraum aufgenommen ist. Mit einem derartigen Aufbau wird ein insgesamt kompakter Aufbau erzielt, der vielfältigste Anordnungsmöglichkeiten im Gehäuseinnenraum sowie dessen vorteilhafte Unterteilung ermöglicht. Der untere Gehäuseschalenbereich der inneren Gehäuseschale muss dabei nicht als geschlossener, kuppelartiger Gehäuseschalenbereich ausgebildet sein, sondern könnte auch mit wenigstens einer Ausnehmung oder Aussparung versehen sein, zum Beispiel im Bereich seiner Seitenwand und/oder seiner Deckenwand.

Des Weiteren ist die innere Gehäuseschale entweder integraler Bestandteil der Außenschale bzw. einer der Außenschalen oder durch ein separates Bauteil gebildet. Alternativ oder zusätzlich kann die innere Gehäuseschale den Fingereingriffsraum und den Aufnahmeraum für das Behältnis auch vom restlichen Gehäuseinnenraum abtrennen, so zum Beispiel von dem die Steuerungselektronik aufnehmenden Gehäuseinnenraum abtrennen, so dass auf einfache Weise sichergestellt ist, dass bei einem Fingereingriff kein Zugriff in nicht erwünschte bzw. nicht freigegebene Bereiche des Gehäuseinnenraums erfolgt.

Gemäß einer weiteren besonders bevorzugten Ausgestaltung ist vorgesehen, dass eine durch einen gehäuseseitigen Schalter oder durch eine gehäuseseitige Taste aktivierbare Steuerungselektronik vorgesehen ist, die wenigstens eine im Gehäuseinnenraum aufgenommene Leiterplatte aufweist und mittels der das piezoelektrisch betriebene Zerstäubersystem, insbesondere das Piezoelement des Zerstäubersystems, ansteuerbar ist. Damit kann auf einfache Weise von der Außenseite des Gehäuses her der Dispenser bzw. das piezoelektrisch betriebene Zerstäubersystem betätigt werden.

Die Erfindung wird nachfolgend anhand einer Zeichnung beispielhaft näher erläutert.

Es zeigen:
- Fig. 1: Eine schematische perspektivische Draufsicht auf eine beispielhafte Ausführungsform eines erfindungsgemäßen piezoelektrischen Dispensers,
- Fig. 2: der Dispenser gemäß Fig. 1 ohne obere Gehäuseschale,
- Fig. 3: eine schematische Explosionsdarstellung des Dispensers gemäß Fig. 1 und 2,
- Fig. 4: eine schematische, vergrößerte Detailansicht eines Teilbereichs der Schnappverbindung ohne eingesetztes Behältnis,
- Fig. 5: eine der Fig. 4 entsprechende vergrößerte Detaildarstellung mit eingesetztem Behältnis,
- Fig. 6: eine schematische Schnittdarstellung durch den Dispenser mit eingesetztem Behältnis und zwei Fingereingriffsöffnungen mitsamt Fingereingriffsräumen,
- Fig. 7a bis 7c: verschiedene Unteransichten des Dispensers mit verschiedenen Einsetzzuständen des Behälters.

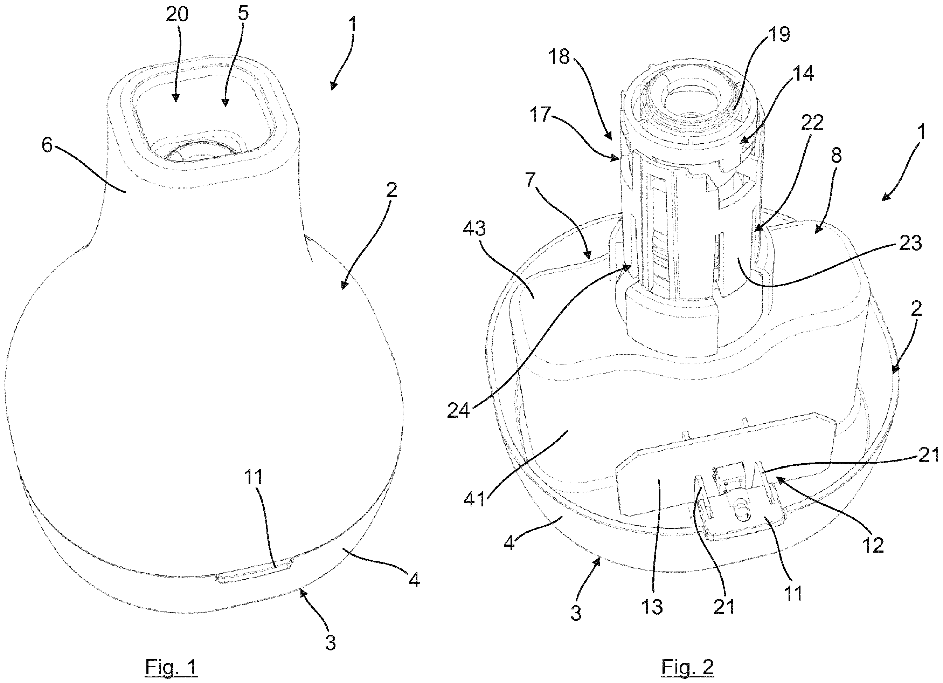

In der Fig. 1 ist schematisch eine perspektivische Ansicht einer beispielhaften Ausführungsform eines erfindungsgemäßen piezoelektrisch betriebenen Dispensers 1 gezeigt, dessen Gehäuse 2 hier durch eine zweiteilige Außenschale mit einer, bezogen auf die Hochachsenrichtung, unteren, den Boden- oder Aufstandsbereich 3 ausbildenden Gehäuseaußenschale 4 und einer damit verbundenen oberen, eine Auslassöffnung 5 aufweisenden Gehäuseaußenschale 6 gebildet ist.

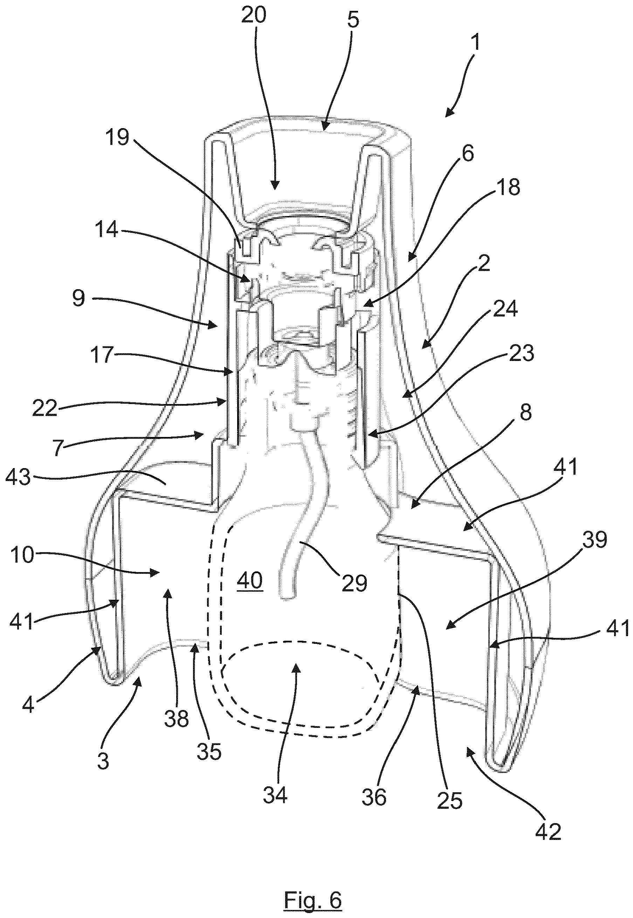

In der Fig. 2 ist die obere Gehäuseaußenschale 6 weggelassen und somit der Blick auf einen Gehäuseinnenraum 7 freigegeben, in dem eine innere Gehäuseschale 8 angeordnet, die den Gehäuseinnenraum 7, wie dies insbesondere aus der Fig. 6 ersichtlich ist, in einen, bezogen auf die Hochachsenrichtung oberen Innenraumbereich 9 und einen unteren Innenraumbereich 10 unterteilt.

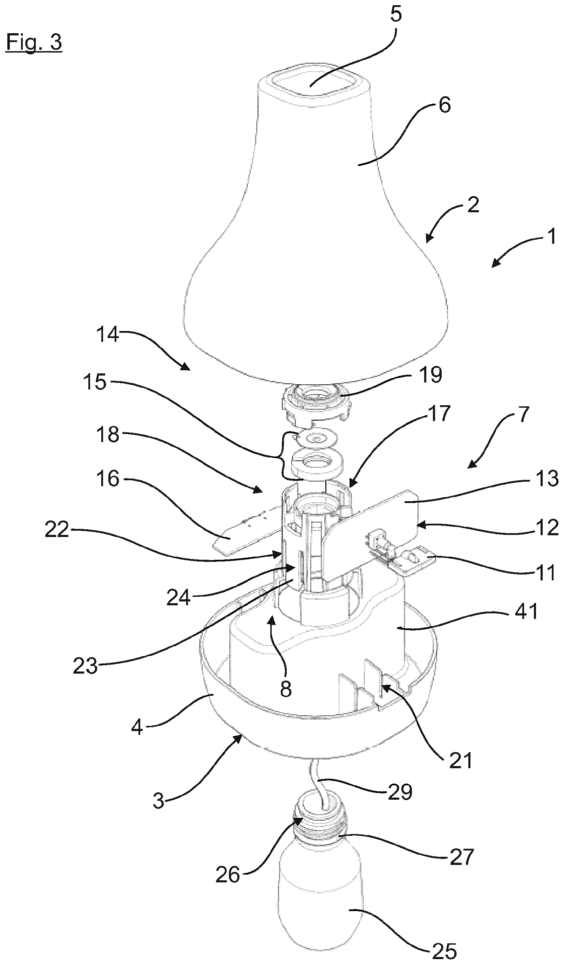

Zwischen der unteren Gehäuseaußenschale 4 und der oberen Gehäuseschale 6 ist hier lediglich beispielhaft eine gehäuseseitig gelagerte Taste 11 vorgesehen, mit der eine Steuerungselektronik 12 aktivierbar bzw. deaktivierbar ist. Diese Steuerungselektronik 12 weist eine im Gehäuseinnenraum aufgenommene Leiterplatte 13 auf, mittels der ein piezoelektrisch betriebenes Zerstäubersystem 14 (siehe auch Fig. 3), insbesondere ein elektrisches Piezoelement 15 des Zerstäubersystems 14, ansteuerbar ist, was hier allerdings ebensowenig im Detail dargestellt ist wie die Stromversorgung der Steuerungselektronik 12 über wenigstens eine Batterie und/oder über einen externen Stromanschluss. Beispielsweise kann die Stromversorgung über ein Stromkabel oder bevorzugt über einen Micro-USB-Anschluss erfolgen, zum Beispiel über die ebenfalls im Gehäuseinnenraum 7 aufgenommene Power-Leiterplatte 16 und einen hier nicht dargestellten, jedoch gegenüberliegend zur Taste 11 an der unteren Gehäuseaußenschale 4 angeordneten Micro-USB-Anschluss.

Wie dies insbesondere aus der Fig. 2 ersichtlich ist, ist die Leiterplatte 13 in eine Steckaufnahme 21 in der unteren Gehäuseaußenschale 4 eingesteckt. Gleiches gilt für die Power-Leiterplatte 16 auf der gegenüberliegenden Seite.

Das Piezoelement 15 ist in einem röhrenförmig ausgebildeten Dom 17 der inneren Gehäuseschale 8 angeordnet, und zwar in einem, bezogen auf die Hochachsenrichtung, oberen Teilbereich 18 des Doms 17, auf den endseitig ein Piezo-Gehäuse 19 aufgesetzt ist. Wie dies insbesondere aus der Fig. 6 ersichtlich ist, schließt sich an dieses Piezo-Gehäuse 19 in Hochachsenrichtung nach oben ein die Auslassöffnung 5 ausbildender Auslasskanal 20 an. Über diesen Auslasskanal 20 bzw. die Auslassöffnung 5 gelangt die mittels des Piezoelementes 15 zerstäubte Substanz aus dem Dispenser 1 vorzugsweise als feiner Sprühnebel in die Umgebung.

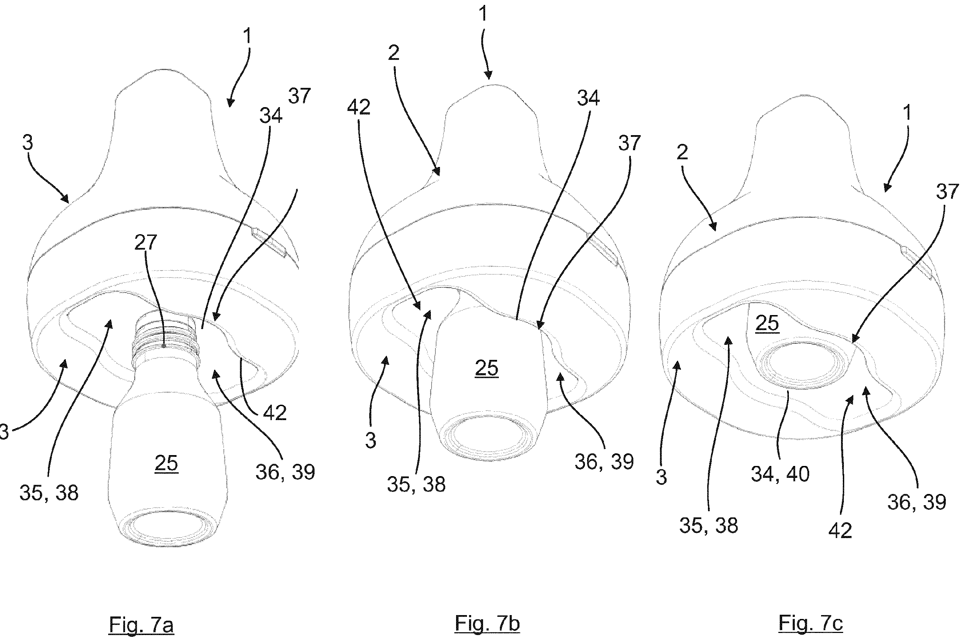

Wie dies aus der Zusammenschau der Fig. 2, 3 und 6 weiter ersichtlich ist, weist der Dom 17 ferner einen sich in Hochachsenrichtung gesehen nach unten an den oberen Teilbereich 18 anschließenden unteren Teilbereich 22 auf, an dem eine Mehrzahl von in Dom-Umfangsrichtung voneinander beabstandeten Schnapphaken 23 einer Schnappverbindung 24 ausgebildet sind. Mittels dieser Schnappverbindung 24 kann ein Behältnis 25 lösbar im Gehäuseraum 7 in nachstehend noch näher beschriebener Weise gehaltert werden, und zwar bevorzugt dergestalt, dass dieses Behältnis 25, wie in der Fig. 6 und auch in der Fig. 7c gezeigt, vollständig im Gehäuseinnenraum 7 aufgenommen ist.

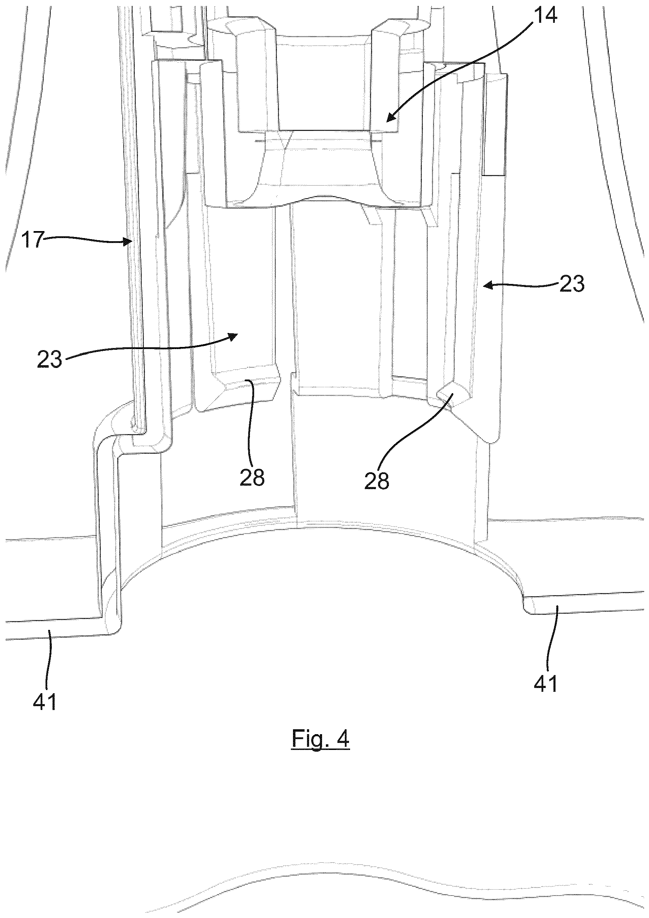

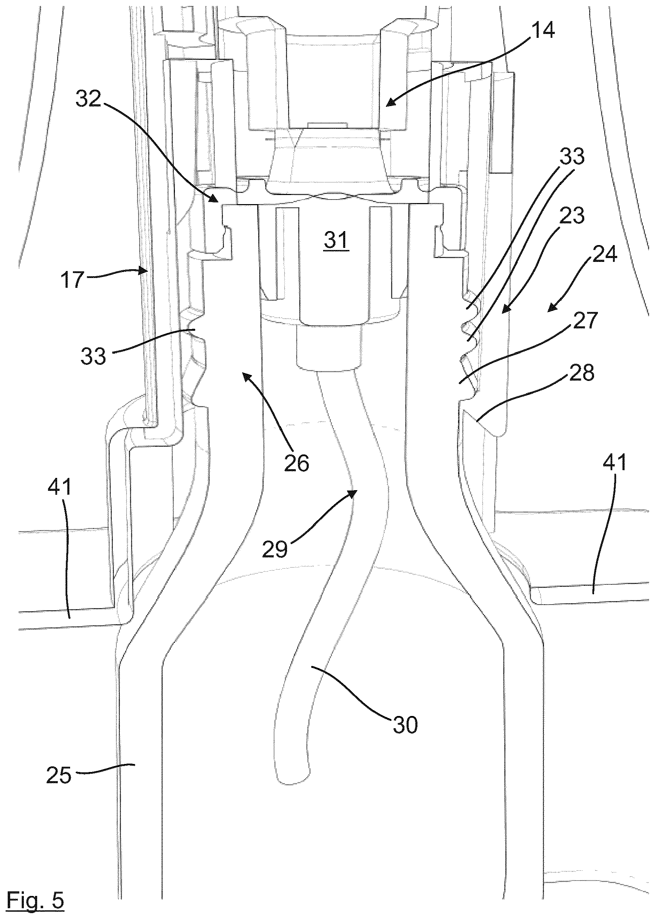

Die Schnappverbindung 24 wird nachstehend in Verbindung mit den Detaildarstellung der Fig. 4 und 5 näher erläutert:
Wie dies aus der Fig. 5 ersichtlich ist, weist das Behältnis 25 vorzugweise einen in der Art eines Rohrstutzens bzw. in der Art eines Flaschenhalses ausgebildeten Auslassöffnungsbereich 26 auf, der an seiner Außenseite einen hier beispielhaft ringförmig umlaufenden Wulst 27 als Haltebereich aufweist. Wie dies aus den Darstellungen der Fig. 4 und 5 weiter ersichtlich ist, sind die Schnapphaken 23, im eingesetzten Zustand des Behältnisses 25 (Fig. 5) ringförmig und voneinander beabstandet, um den Auslassöffnungsbereich 26 des Behältnisses 25 herum angeordnet und hintergreifen mit ihren jeweiligen Rastvorsprüngen 28 den ringförmigen Wulst 27.

Konkret sind die Schnapphaken 23 hier als elastisch rückfedernde Schnapphaken 23 ausgebildet, zum Beispiel aus einem Kunststoffmaterial, um nur ein Beispiel zu nennen, sodass die Schnapphaken 23 beim Einführen des Behältnisses 25 kurzzeitig ausgelenkt werden und dann am Ende des Montagevorgangs wieder in die in der Fig. 5 gezeigte Stellung einrasten und den Wulst 27 hintergreifen. Die Haltekraft der Schnapphaken 23 wird dabei insbesondere durch die Geometrie der Rastvorsprünge 28 der Schnapphaken 23 vorgegeben. Beim Herausnehmen des Behältnisses wird dann das Behältnis 25 mit einer entsprechenden Zugkraft, in Hochachsenrichtung gesehen, nach unten gezogen, wodurch die Schnappverbindung 24 überdrückt wird, da die Schnapphaken 23 durch den Wulst 27 wieder entsprechend elastisch ausgelenkt werden und dadurch deren Rastvorsprünge 28 außer Eingriff mit dem Wulst 27 des Auslassöffnungsbereichs 26 des Behältnisses 25 gelangen.

Wie dies aus der Fig. 5 weiter ersichtlich ist, weist das Zerstäubersystem 14 weiter einen Docht 29 als Zuführeinrichtung auf, mittels dem die in dem Behältnis 25 aufgenommene Substanz dem Piezoelement 15 zuführbar ist. Konkret weist der Docht 29 einen ersten Teilbereich 30 auf, der in die Substanz einragt (Fig. 5), während ein gegenüberliegendes freies Ende 29 des Dochtes 29 an das Piezoelement 15 angrenzt und dieses mit der flüssigen Substanz versorgt.

Im in der Fig. 5 gezeigten Beispielfall ist der Docht 29 mittels einer Halteeinrichtung 32 am oberen freien Ende des Auslassöffnungsbereichs 26 gehaltert.

Ferner ist aus der Fig. 5 noch weiter ersichtlich, dass am Auslassöffnungsbereich 26 ein Gewinde 33 angeordnet ist, das dazu dient, das Behältnis 25, zum Beispiel im Nicht-Gebrauchszustand des Behältnisses 25, mit einer hier nicht dargestellten Gewindekappe zu verschließen.

Wie dies nunmehr weiter aus der Zusammenschau der Fig. 6, 7a, 7b und 7c ersichtlich ist, weist der Boden- und Aufstandsbereich 3 des Gehäuses 2 des piezoelektrisch betriebenen Dispensers 1 hier eine Einführöffnung 34 für das Behältnis 25 auf, über die das Behältnis 25 in den Gehäuseinnenraum 7 bzw. einen nachstehend noch näher erläuterten Behältnis-Aufnahmeraum 40 einführbar ist. Die Einführöffnung 34 liegt hier in etwa mittig zwischen zwei sich daran zu beiden bzw. auf gegenüberliegenden Seiten hin anschließenden Fingereingriffsöffnungen 35, 36, sodass die Einführöffnung 34 mitsamt den Fingereingriffsöffnungen 35, 36 hier beispielhaft eine langestreckte Schlitzausnehmung 42 ausbildet. Diese Schlitzausnehmung 42 weist in dem zwischen den beiden Fingereingriffsöffnungen 35, 36 liegenden und die Einführöffnung 34 für das Behältnis 25 ausbildenden Ausnehmungsbereich eine dem Außenumfang des Behältnisses 25 entsprechende Verbreiterung 37 auf, die es dem Bediener erleichtert, die exakte Positionierung des Behältnisses 25 beim Einsetzen des Behältnisses 25 zu finden. Die Verbreiterung 37 ist hier nur ein Beispiel. Alternativ sind auch andere Geometrien der Schlitzausnehmung möglich, die dann beispielsweise anstelle einer Verbreiterung eine Markierung aufweisen oder eine Einschnürung aufweisen. Auch eine Verbreiterung mitsamt Markierung oder eine Einschnürung mitsamt Markierung ist selbstverständlich möglich.

Es versteht sich, dass selbstverständlich auch andere Schlitzausnehmungsgeometrien jederzeit möglich sind, zum Beispiel dergestalt, dass die Einführöffnung bezogen auf die sich daran auf gegenüberliegenden Seiten anschließenden Eingriffsöffnungen außermittig angeordnet ist, um nur ein Beispiel zu nennen.

Wie dies weiter insbesondere aus der Fig. 6 ersichtlich ist, gehen die zwei FingerEingriffsöffnungen 35, 36 zum Gehäuseinnenraum 7 hin in einen Fingereingriffsraum 38, 39 über bzw. bilden die Fingereingriffsräume 38, 39 Bestandteil der FingerEingriffsöffnungen 35, 36.

Diese beiden Fingereingriffsräume 38, 39 und der sich an die Einführöffnung 34 anschließende Behältnis-Aufnahmeraum 40 bilden den hier durch die innere Gehäuseschale 8 vom oberen Innenraumbereich 9 abgetrennten unteren Innenraumbereich 10 aus. Wie dies ebenfalls wiederum aus der Zusammenschau der Fig. 6, 7a, 7b und 7c ersichtlich ist, schließt sich hierzu die innere Gehäuseschale 8, der Geometrie der Schlitzausnehmung 42, folgend mit einem unteren Gehäuseschalenbereich 41 turmartig an die Schlitzausnehmung 42 an. Dieser untere Gehäuseschalenbereich 41 erstreckt sich dabei hier beispielhaft so weit in Hochachsenrichtung nach oben, dass sichergestellt ist, dass das Behältnis 25 im eingesetzten Zustand im Wesentlichen vollständig im Gehäuseinnenraum 7 bzw. im Behältnis-Aufnahmeraum 40 aufgenommen ist. Von diesem unteren Gehäuseschalenbereich 41 ausgehend bzw. konkret von einer Deckenwand 43 des unteren Gehäuseschalenbereiches 41 ausgehend, erstreckt sich dann der Dom 17 in Hochachsenrichtung gesehen nach oben.

Der untere Gehäuseschalenbereich 41 der inneren Gehäuseschale 8 muss nicht, wie im Ausführungsbeispiel dargestellt, als geschlossener, kuppelartiger Gehäuseschalenbreich ausgebildet sein, sondern könnte auch mit wenigstens einer Ausnehmung oder Aussparung versehen sein, zum Beispiel im Bereich seiner Seitenwand und/oder seiner Deckenwand 43, was hier aber nicht mehr dargestellt ist.

Zum Einführen des Behältnisses 25 in den Gehäuseinnenraum 7 kann, wie dies in der Fig. 7a lediglich schematisch dargestellt ist, das Behältnis 25 im Bereich der Einführöffnung 34 positioniert und dort beispielsweise mit dem Daumen und Zeigefinger einer Hand (nicht dargestellt) gehalten werden. Anschließend wird dann (Fig. 7b) das Behältnis solange in den Gehäuseinnenraum 7 eingeführt, bis der Auslassöffnungsbereich 26 des Behältnisses 25 sicher mittels der Schnappverbindung 24 von den Schnapphaken 23 hintergriffen ist (Fig. 7c). Dabei greifen die Finger durch die Fingereingriffsöffnungen 35, 36 hindurch mit in den Gehäuseinnenraum 7 ein, und zwar konkret in die Fingereingriffsräume 38, 39, die hier lediglich beispielsweise mittels der inneren Gehäuseschale 8 vom restlichen Gehäuseinnenraum abgeschirmt sind.

Das Herausnehmen des Behältnisses 25 erfolgt in analoger Weise umgekehrt, indem zwei Finger einer Hand (nicht dargestellt) durch die Fingereingriffsöffnungen 35, 36 hindurch in die Fingereingriffsräume 38 und 39 eingeführt werden, um das Behältnis 25 im Gehäuseinnenraum 7 umfangsseitig zu ergreifen und nach unten zu ziehen, wodurch die Schnappverbindung 34 ausrastet und gelöst wird.

### Bezugszeichenliste

- 1: Dispenser
- 2: Gehäuse
- 3: Bodenbereich
- 4: untere Gehäuseaußenschale
- 5: Auslassöffnung
- 6: obere Gehäuseaußenschale
- 7: Gehäuseinnenraum
- 8: innere Gehäuseschale
- 9: oberer Innenraumbereich
- 10: unterer Innenraumbereich
- 11: Taste
- 12: Steuerungselektronik
- 13: Leiterplatte
- 14: Zerstäubersystem
- 15: Piezoelement
- 16: Power-Leiterplatte
- 17: Dom
- 18: oberer Teilbereich des Domes
- 19: Piezo-Gehäuse
- 20: Auslasskanal
- 21: Steckaufnahme
- 22: unterer Teilbereich des Domes
- 23: Schnapphaken
- 24: Schnappverbindung
- 25: Behältnis
- 26: Auslassöffnungsbereich
- 27: Wulst
- 28: Rastvorsprung
- 29: Docht
- 30: erster Teilbereich
- 31: freies Ende
- 32: Halteeinrichtung
- 33: Gewinde
- 34: Einführöffnung
- 35: Fingereingriffsöffnung
- 36: Fingereingriffsöffnung
- 37: Verbreiterung
- 38: Fingereingriffsraum
- 39: Fingereingriffsraum
- 40: Behältnis-Aufnahmeraum
- 41: unterer Gehäuseschalenbereich
- 42: Schlitzausnehmung
- 43: Deckenwand

## Patentansprüche

1. Piezoelektrisch betriebener Dispenser (1) zum Austragen flüssiger oder flüchtiger Substanzen,
mit einem Gehäuse (2), das einen Gehäuseinnenraum (7) aufweist, wobei das Gehäuse (2) eine ein- oder mehrteilige Außenschale aufweist, die den Gehäuseinnenraum (7) nach außen hin begrenzt, einen Boden- oder Aufstandsbereich (3) ausbildet und wenigstens eine Auslassöffnung (5) für die abzugebende Substanz aufweist,
mit einem Behältnis (25), das lösbar mit dem Gehäuse (2) verbindbar ist und in dem eine auszutragende und/oder zu verflüchtigende Substanz aufgenommen ist, und
mit einem piezoelektrisch betriebenen Zerstäubersystem (14), das in dem Gehäuseinnenraum (7) angeordnet ist und mittels dem die aus dem Behältnis (25) entnommene Substanz zerstäubt wird,
wobei das Behältnis (25) im montierten Zustand im Gehäuseinnenraum (7) aufgenommen und dort mittels wenigstens einer Schnappverbindung (24) lösbar gehaltert ist,
wobei das Gehäuse (2) eine Einführöffnung (34) für das Behältnis (25) aufweist, über die das Behältnis (25) in den Gehäuseinnenraum (7) einführbar ist,
wobei das Gehäuse (2) im Bereich der Einführöffnung (34) wenigstens eine Fingereingriffsöffnung (35, 36) aufweist, dergestalt, dass beim Einführen des Behältnisses (25) in den Gehäuseinnenraum (7) und beim Entnehmen des Behältnisses (25) aus dem Gehäuseinnenraum (7) ein Fingereingriff in den Gehäuseinnenraum (7) möglich ist,
**dadurch gekennzeichnet,**
**dass** die Einführöffnung (34) und zwei Fingereingriffsöffnungen (35, 36) in dem Boden- oder Aufstandsbereich (3) der Außenschale des Gehäuses (2) ausgebildet sind,
**dass** die Einführöffnung (34) in einem mittleren Bereich zwischen den zwei sich daran auf gegenüberliegenden Seiten hin anschließenden Fingereingriffsöffnungen (35, 36) liegt.

2. Dispenser nach Anspruch 1, **dadurch gekennzeichnet, dass** die Schnappverbindung (24) durch wenigstens einen, im Gehäuseinnenraum (7) elastisch rückfedernd angeordneten Schnapphaken (23) gebildet ist, der wenigstens einen, vorzugsweise endseitig angeordneten, Rastvorsprung (28) aufweist, der bei einem in das Gehäuse (2) eingesetzten, montierten Behältnis (25) einen behältnisseitigen Haltebereich hintergreift oder in einen behältnisseitigen Haltebereich eingreift, so dass das Behältnis (25) lösbar im Gehäuseinnenraum (7) gehaltert ist.

3. Dispenser nach Anspruch 1 oder 2, **dadurch gekennzeichnet, dass** das Behältnis (25) einen in der Art eines Rohrstutzens und/oder in der Art eines Flaschenhalses ausgebildeten Auslassöffnungsbereich (26) aufweist, der an seiner Außenseite einen wenigstens abschnittsweise, vorzugsweise ringförmig, umlaufenden Wulst (27) oder eine wenigstens abschnittsweise, vorzugsweise ringförmig, umlaufende Nut als Haltebereich aufweist.

4. Dispenser nach Anspruch 2 und 3, **dadurch gekennzeichnet, dass** im Gehäuseinnenraum (7) eine Mehrzahl von Schnapphaken (23) vorgesehen ist, die im eingesetzten Zustand des Behältnisses (25) ringförmig und voneinander beabstandet um den Auslassöffnungsbereich (26) des Behältnisses (25) herum angeordnet sind und mit ihren jeweiligen Rastvorsprüngen (28) den Wulst (27) hintergreifen oder in die Nut eingreifen.

5. Dispenser nach einem der vorhergehenden Ansprüche, **dadurch gekennzeichnet, dass** im Gehäuseinnenraum (7) eine innere Gehäuseschale (8) vorgesehen ist, die einen im Gehäuseinnenraum (7) ausgebildeten Aufnahmeraum (40) für das Behältnis (25) ausbildet, insbesondere begrenzt, und/oder an der der gehäuseseitige Teilbereich der Schnappverbindung (24) angeordnet und/oder ausgebildet ist, vorzugsweise die Schnapphaken (23) der Schnappverbindung (24) angeordnet und/oder ausgebildet sind.

6. Dispenser nach einem der vorhergehenden Ansprüche, **dadurch gekennzeichnet,**
**dass** das Zerstäubersystem (14) ein elektrisch betätigbares Piezoelement (15) aufweist, und
**dass** das Zerstäubersystem (15) eine Zuführeinrichtung aufweist, mittels der die in dem Behältnis (25) aufgenommene Substanz dem Piezoelement (15) zuführbar ist.

7. Dispenser nach Anspruch 5 und 6, **dadurch gekennzeichnet,**
**dass** die innere Gehäuseschale (8) einen sich in Hochachsenrichtung von dem eingesetzten, montierten Behältnis (25) weg erstreckenden Dom (17) aufweist, der mit seinem unteren Teilbereich (22) den gehäuseseitigen Teilbereich der Schnappverbindung (24) ausbildet, vorzugsweise die Schnapphaken (23) der Schnappverbindung (24) ausbildet,
**dass** das Piezoelement (15) in einem sich an den unteren Teilbereich des Doms anschließenden oberen Teilbereich (18) des Doms (17) angeordnet und/oder aufgenommen ist, und
**dass** das Piezoelement (15) in einen, in Hochachsenrichtung gesehen, oberen Gehäusebereich des Gehäuses (2) mündet, der eine Auslassöffnung (5), bevorzugt einen Auslasskanal (20), höchst bevorzugt einen in der Art eines Kamins ausgebildeten Auslasskanal, für die vom Piezoelement (15) zerstäubte, abzugebende Substanz aufweist und/oder ausbildet.

8. Dispenser nach Anspruch 6 oder 7, **dadurch gekennzeichnet, dass** die Zuführeinrichtung durch einen Docht (29) gebildet ist, der im eingesetzten, montierten Zustand des Behältnisses (25) mit einem ersten Teilbereich in die im Behältnis (25) aufgenommene Substanz einragt und mit einem gegenüberliegenden freien Ende an das Piezoelement (15) angrenzt, vorzugsweise aus dem Behältnis (25) ragt und an das Piezoelement (15) angrenzt.

9. Dispenser nach einem der vorhergehenden Ansprüche, **dadurch gekennzeichnet, dass** das Gehäuse (2) eine zweiteilige Außenschale mit einer, bezogen auf die Hochachsenrichtung, unteren, den Boden- oder Aufstandsbereich (3) ausbildenden Gehäuseaußenschale (4) und einer damit verbundenen oberen, die wenigstens eine Auslassöffnung (5) aufweisenden Gehäuseaußenschale (6) aufweist.

10. Dispenser nach einem der vorhergehenden Ansprüche, **dadurch gekennzeichnet, dass** die Einführöffnung (34) mitsamt den zwei Fingereingriffsöffnungen (35, 36) eine langgestreckte Schlitzausnehmung (42) ausbildet.

11. Dispenser nach Anspruch 10, **dadurch gekennzeichnet, dass** die Schlitzausnehmung (42) in dem zwischen den beiden Fingereingriffsöffnungen (35, 36) liegenden und die Einführöffnung (34) für das Behältnis (25) ausbildenden Ausnehmungsbereich eine Markierung aufweist.

12. Dispenser nach einem der vorhergehenden Ansprüche, **dadurch gekennzeichnet, dass** die gehäuseseitig ausgebildeten Fingereingriffsöffnungen (35, 36) zum Gehäuseinnenraum (7) hin in einen Fingereingriffsraum (38, 39) übergeht.

13. Dispenser nach einem der Ansprüche 10 bis 12, **dadurch gekennzeichnet, dass** sich die innere Gehäuseschale (8), der Geometrie der Schlitzausnehmung (42) folgend, mit einem unteren Gehäuseschalenbereich (41) turmartig an die Schlitzausnehmung (42) anschließt und gleichzeitig den Fingereingriffsraum (38, 39) sowie den Behältnis-Aufnahmeraum (40) ausbildet, wobei bevorzugt vorgesehen ist, dass sich der untere Gehäuseschalenbereich (41) der inneren Gehäuseschale (8) so weit in Hochachsenrichtung nach oben erstreckt, dass das Behältnis (25) im eingesetzten Zustand im Wesentlichen vollständig im Gehäuseinnenraum (7) und/oder im Behältnis-Aufnahmeraum (40) aufgenommen ist.

14. Dispenser nach einem der Ansprüche 5 bis 13, **dadurch gekennzeichnet, dass** die innere Gehäuseschale (8) integral mit der Außenschale verbunden ist oder durch ein separates Bauteil gebildet ist und/oder dass die innere Gehäuseschale (8) den Fingereingriffsraum (38, 39) und den Behältnis-Aufnahmeraum (40) vom restlichen Gehäuseinnenraum abtrennt.

15. Dispenser nach einem der vorhergehenden Ansprüche, **dadurch gekennzeichnet, dass** eine durch einen gehäuseseitigen Schalter oder durch eine gehäuseseitige Taste (11) aktivierbare Steuerungselektronik (12) vorgesehen ist, die wenigstens eine im Gehäuseinnenraum (7) aufgenommene Leiterplatte (16) aufweist und mittels der das piezoelektrisch betriebene Zerstäubersystem (14), insbesondere das Piezoelement (15) des Zerstäubersystems (14), ansteuerbar ist.

## Claims

1. Piezoelectrically operated dispenser (1) for dispensing liquid or volatile substances,
with a housing (2) which has a housing interior (7), the housing (2) having a single-part or multi-part outer shell which delimits the housing interior (7) towards the outside, forms a base- or support-area (3) and has at least one outlet opening (5) for the substance to be dispensed,
with a container (25) which is detachably connectable to the housing (2) and in which a substance to be discharged and/or volatilised is received, and
with a piezoelectrically operated atomiser system (14), which is arranged in the housing interior (7) and by means of which the substance removed from the container (25) is atomised,
wherein the container (25) is received in the housing interior (7) in the assembled state and is releasably held there by means of at least one snap connection (24),
wherein the housing (2) has an insertion opening (34) for the container (25), via which the container (25) is insertable into the housing interior (7),
wherein the housing (2) has at least one finger engagement opening (35, 36) in the region of the insertion opening (34) in such a way that finger engagement in the housing interior (7) is possible when the container (25) is inserted into the housing interior (7) and when the container (25) is removed from the housing interior (7),
**characterised in that**
the insertion opening (34) and two finger engagement openings (35, 36) are formed in the base- or support-area (3) of the outer shell of the housing (2),
that the insertion opening (34) is located in a central region between the two finger engagement openings (35, 36) adjoining it on opposite sides.

2. Dispenser according to claim 1, **characterised in that** the snap connection (24) is formed by at least one snap hook (23) which is arranged in the housing interior (7) in an elastically resilient manner and which has at least one latching projection (28), preferably arranged at the end, which, when a mounted container (25) is inserted into the housing (2), engages behind a container-side retaining region or engages in a container-side retaining region, so that the container (25) is releasably retained in the housing interior (7).

3. Dispenser according to claim 1 or 2, **characterised in that** the container (25) has an outlet opening region (26) formed in the manner of a pipe socket and/or in the manner of a bottle neck, which has on its outside an at least sectionally, preferably annularly, encircling bead (27) or an at least sectionally, preferably annularly, encircling groove as a retaining region.

4. Dispenser according to claims 2 and 3, **characterised in that** a plurality of snap hooks (23) are provided in the housing interior (7), which, in the inserted state of the container (25), are arranged annularly and spaced apart from one another around the outlet opening region (26) of the container (25) and engage behind the bead (27) with their respective latching projections (28) or engage in the groove.

5. Dispenser according to one of the preceding claims, **characterised in that** an inner housing shell (8) is provided in the housing interior (7), which forms, in particular delimits, a receiving space (40) formed in the housing interior (7) for the container (25), and/or on which the housing-side partial region of the snap connection (24) is arranged and/or formed, preferably the snap hooks (23) of the snap connection (24) are arranged and/or formed.

6. Dispenser according to one of the preceding claims, **characterised in that,**
the atomiser system (14) has an electrically actuable piezo element (15), and
**in that** the atomiser system (15) has a feed device by means of which the substance contained in the container (25) is feedable to the piezo element (15).

7. Dispenser according to claims 5 and 6, **characterised in that,**
the inner housing shell (8) has a dome (17) which extends away from the inserted, mounted container (25) in the vertical axis direction and which forms the housing-side sub-region of the snap connection (24) with its lower sub-region (22), preferably forming the snap hooks (23) of the snap connection (24),
**in that** the piezo element (15) is arranged and/or received in an upper subregion (18) of the dome (17) adjoining the lower subregion of the dome, and
**in that** the piezo element (15) opens into an upper housing region of the housing (2), viewed in the vertical axis direction, which has and/or forms an outlet opening (5), preferably an outlet channel (20), most preferably an outlet channel formed in the manner of a chimney, for the substance to be discharged which is atomised by the piezo element (15).

8. Dispenser according to claim 6 or 7, **characterised in that** the feed device is formed by a wick (29) which, in the inserted, mounted state of the container (25), projects with a first partial region into the substance received in the container (25) and adjoins the piezo element (15) with an opposite free end, preferably projects out of the container (25) and adjoins the piezo element (15).

9. Dispenser according to one of the preceding claims, **characterised in that** the housing (2) has a two-part outer shell with a lower housing outer shell (4), relative to the vertical axis direction, forming the base or support area (3), and an upper housing outer shell (6) connected thereto and having at least one outlet opening (5).

10. Dispenser according to one of the preceding claims, **characterised in that** the insertion opening (34) together with the two finger engagement openings (35, 36) forms an elongated slot recess (42).

11. Dispenser according to claim 10, **characterised in that** the slot recess (42) has a marking in the recess region located between the two finger engagement openings (35, 36) and forming the insertion opening (34) for the container (25).

12. Dispenser according to one of the preceding claims, **characterised in that** the finger engagement openings (35, 36) formed on the housing side merge into a finger-engaging space (38, 39) towards the housing interior (7).

13. Dispenser according to one of claims 10 to 12, **characterised in that** the inner housing shell (8), following the geometry of the slot recess (42), adjoins the slot recess (42) in a tower-like manner with a lower housing shell region (41) and simultaneously forms the fingerengaging space (38, 39) and the container-receiving space (40), wherein it is preferably provided that the lower housing shell region (41) of the inner housing shell (8) extends so far upwards in the vertical axis direction that the container (25) is essentially completely received in the housing interior (7) and/or in the container receiving space (40) in the inserted state.

14. Dispenser according to one of claims 5 to 13, **characterised in that** the inner housing shell (8) is integrally connected to the outer shell or is formed by a separate component and/or **in that** the inner housing shell (8) separates the finger-engaging space (38, 39) and the container-receiving space (40) from the rest of the housing interior.

15. Dispenser according to one of the preceding claims, **characterised in that** an electronic control unit (12) is provided which is activatable by a housing-side switch or by a housing-side button (11) and which has at least one printed circuit board (16) accommodated in the housing interior (7) and by means of which the piezoelectrically operated atomiser system (14), in particular the piezoelectric element (15) of the atomiser system (14), is actuatable.

## Revendications

1. Distributeur à commande piézoélectrique (1) pour la distribution de substances liquides ou volatiles,
avec un boîtier (2) ayant un intérieur (7), le boîtier (2) ayant une enveloppe extérieure en une ou plusieurs parties qui délimite l'intérieur du boîtier (7) vers l'extérieur, forme une zone de base ou de support (3) et a au moins une ouverture de sortie (5) pour la substance à distribuer,
avec un récipient (25) qui peut être relié de manière amovible au boîtier (2) et dans lequel est reçue une substance à évacuer et/ou à volatiliser, et
avec un système d'atomisation (14) à fonctionnement piézoélectrique, disposé à l'intérieur du boîtier (7) et permettant d'atomiser la substance prélevée dans le récipient (25),
dans lequel le récipient (25) est reçu dans l'intérieur du boîtier (7) à l'état assemblé et y est maintenu de manière amovible au moyen d'au moins un raccord à déclic (24),
dans lequel le boîtier (2) possède une ouverture d'insertion (34) pour le récipient (25), par laquelle le récipient (25) peut être inséré à l'intérieur du boîtier (7),
dans lequel le boîtier (2) comporte au moins une ouverture d'engagement des doigts (35, 36) dans la zone de l'ouverture d'insertion (34) de telle sorte que l'engagement des doigts à l'intérieur du boîtier (7) est possible lorsque le récipient (25) est inséré à l'intérieur du boîtier (7) et lorsque le récipient (25) est retiré de l'intérieur du boîtier (7),
**caractérise en ce que**
l'ouverture d'insertion (34) et deux ouvertures d'engagement des doigts (35, 36) sont formées dans la zone de base ou de support (3) de la coque extérieure du boîtier (2),
l'ouverture d'insertion (34) est située dans une zone centrale entre les deux ouvertures d'engagement des doigts (35, 36) qui la jouxtent sur les côtés opposés.

2. Distributeur selon la revendication 1, **caractérisé en ce que** le raccord à déclic (24) est formé par au moins un mousqueton (23) qui est disposé dans l'intérieur du boîtier (7) de manière élastique et qui présente au moins une saillie de verrouillage (28), de préférence disposée à l'extrémité, qui, lorsqu'un récipient monté (25) est inséré dans le boîtier (2), s'engage derrière une zone de retenue du côté du récipient ou s'engage dans une zone de retenue du côté du récipient, de sorte que le récipient (25) est retenu de manière libérable dans l'intérieur du boîtier (7).

3. Distributeur selon la revendication 1 ou 2, **caractérisé par le fait que** le récipient (25) possède une zone d'ouverture de sortie (26) formée à la manière d'un embout de tuyau et/ou à la manière d'un goulot de bouteille, qui présente à l'extérieur un bourrelet (27) encerclant au moins sectionnellement, de préférence annulairement, ou une rainure encerclant au moins sectionnellement, de préférence annulairement, en tant que zone de retenue.

4. Distributeur selon les revendications 2 et 3, **caractérisé en ce qu'**une pluralité de mousquetons (23) sont prévus à l'intérieur du boîtier (7), qui, à l'état inséré du récipient (25), sont disposés annulairement et à distance les uns des autres autour de la zone d'ouverture de sortie (26) du récipient (25) et s'engagent derrière le bourrelet (27) avec leurs saillies de verrouillage respectives (28) ou s'engagent dans la rainure.

5. Distributeur selon l'une des revendications précédentes, **caractérisé en ce qu'**une coque intérieure (8) est prévue dans l'intérieur du boîtier (7), qui forme, en particulier délimite, un espace de réception (40) formé dans l'intérieur du boîtier (7) pour le récipient (25), et/ou sur lequel la zone partielle côté boîtier du raccord à déclic (24) est disposée et/ou formée, de préférence les mousquetons (23) du raccord à déclic (24) sont disposés et/ou formés.

6. Distributeur selon l'une des revendications précédentes, **caractérisé en ce que,**
le système d'atomisation (14) comporte un élément piézoélectrique (15) pouvant être actionné électriquement, et
le système d'atomisation (15) comporte un dispositif d'alimentation au moyen duquel la substance contenue dans le récipient (25) peut être acheminée vers l'élément piézoélectrique (15).

7. Distributeur selon les revendications 5 et 6, **caractérisé en ce que,**
la coque intérieure (8) comporte un dôme (17) qui s'étend à l'écart du récipient (25) inséré et monté dans la direction de l'axe vertical et qui forme la sous-région côté boîtier du raccord à déclic (24) avec sa sous-région inférieure (22), formant de préférence les mousquetons (23) du raccord à déclic (24),
l'élément piézoélectrique (15) est disposé et/ou reçu dans une sous-région supérieure (18) du dôme (17) adjacente à la sous-région inférieure du dôme, et
l'élément piézoélectrique (15) débouche dans une zone supérieure du boîtier (2), vue dans la direction de l'axe vertical, qui présente et/ou forme une ouverture de sortie (5), de préférence un canal de sortie (20), de préférence encore un canal de sortie formé à la manière d'une cheminée, pour la substance à évacuer qui est atomisée par l'élément piézoélectrique (15).

8. Distributeur selon la revendication 6 ou 7, **caractérisé en ce que** le dispositif d'alimentation est formé par une mèche (29) qui, à l'état inséré et monté du récipient (25), fait saillie avec une première zone partielle dans la substance reçue dans le récipient (25) et jouxte l'élément piézoélectrique (15) avec une extrémité libre opposée, de préférence fait saillie hors du récipient (25) et jouxte l'élément piézoélectrique (15).

9. Distributeur selon l'une des revendications précédentes, **caractérisé en ce que** le boîtier (2) a une coque extérieure en deux parties avec une coque extérieure inférieure (4), par rapport à la direction de l'axe vertical, formant la base ou la zone de support (3), et une coque extérieure supérieure (6) reliée à celle-ci et ayant au moins une ouverture de sortie (5).

10. Distributeur selon l'une des revendications précédentes, **caractérisé en ce que** l'ouverture d'insertion (34) forme, avec les deux ouvertures d'engagement des doigts (35, 36), une fente allongée (42).

11. Distributeur selon la revendication 10, **caractérisé en ce que** l'évidement de la fente (42) présente un marquage dans la zone d'évidement située entre les deux ouvertures d'engagement du doigt (35, 36) et formant l'ouverture d'insertion (34) pour le récipient (25).

12. Distributeur selon l'une des revendications précédentes, **caractérisé en ce que** le fait que les ouvertures d'engagement des doigts (35, 36) formées sur le côté du boîtier se fondent dans un espace d'engagement des doigts (38, 39) vers l'intérieur du boîtier (7).

13. Distributeur selon l'une des revendications 10 à 12, **caractérisé par le fait que** la coque intérieure (8), suivant la géométrie de l'évidement de la fente (42), jouxte l'évidement de la fente (42) en forme de tour avec une zone inférieure de la coque (41) et forme simultanément l'espace d'engagement des doigts (38, 39) et l'espace de réception du récipient (40), dans lequel il est de préférence prévu que la zone inférieure (41) de la coque intérieure (8) s'étende vers le haut dans la direction de l'axe vertical de telle sorte que le récipient (25) soit essentiellement entièrement reçu dans l'intérieur du boîtier (7) et/ou dans l'espace de réception du récipient (40) à l'état inséré.

14. Distributeur selon l'une des revendications 5 à 13, **caractérisé en ce que** la coque intérieure (8) est intégralement reliée à la coque extérieure ou est formée par un composant séparé et/ou par le fait que la coque intérieure (8) sépare l'espace d'engagement du doigt (38, 39) et l'espace de réception du récipient (40) du reste de l'intérieur du boîtier.

15. Distributeur selon l'une des revendications précédentes, **caractérisé en ce que** une unité de commande électronique (12) pouvant être activée par un interrupteur côté boîtier ou un bouton côté boîtier (11) et comportant au moins une carte de circuit imprimé (16) logée à l'intérieur du boîtier (7) et permettant d'actionner le système d'atomisation (14) à fonctionnement piézoélectrique, en particulier l'élément piézoélectrique (15) du système d'atomisation (14).
